# EUROPEAN PATENT SPECIFICATION

(11) **EP 4 384 250 B1**
(45) Date of publication and mention of the grant of the patent: **23.09.2026**
(21) Application number: 22764384.8
(22) Date of filing: 09.08.2022
(51) Int. Cl.: A61M 25/00, A61M 25/01, A61M 25/06

(54) **AXIAL ACTUATION MECHANISM FOR CATHETERS**
AXIALER BETÄTIGUNGSMECHANISMUS FÜR KATHETER
MÉCANISME D'ACTIONNEMENT AXIAL POUR CATHÉTERS

(30) Priority: 10.08.2021 EP 21190660; 25.01.2022 EP 22153071
(43) Date of publication of application: 19.06.2024
(73) Proprietor: BIOTRONIK AG, 8180 Bülach (CH)
(72) Inventor: WILLIMANN, Martial, 8418 Schatt (CH); KLAUS, Sebastian, 8424 Embrach (CH)
(74) Representative: WSL Patentanwälte Partnerschaft mbB
(86) International application number: PCT/EP2022/072289
(87) International publication number: WO 2023/017005

(56) References cited:
- EP-A1- 3 758 781
- US-A- 4 846 174
- US-A1- 2020 238 056
- US-A1- 2021 001 096

## Description

The present invention relates to a catheter system for the treatment of vascular (e.g. coronary) or non-vascular conditions or diseases, for example for treating stenoses, occlusions, lesions, for therapeutic purposes as well as for applying drug or fluid media. The catheter system comprises an axial actuation mechanism e.g. for mechanical probing or channeling of stenoses as well as applying drug or media. A method (not being part of the invention) for operating such a catheter system is disclosed. The invention also relates to an axial movement restriction element for restricting an axial movement of at least one inner member being arranged in a lumen of an outer catheter.

Certain clinical interventional procedures, for example related to diagnosis and therapy of stenoses are often demanding when the patient anatomy or vessel morphology is complex. Examples of medical catheter systems can be found in documents US-A-20211001096, US- A-4846174, EP-A-3758781 or US2020/238056.

Thus, it is an object of the present invention to provide a catheter system facilitating several functionalities, such as improved guide-wire negotiations (maneuvering through the vascular system), lesion penetrations and lesion recanalizations as well as application of media or drugs.

This problem is solved by a catheter system having the features of claim 1.

Preferred embodiments are stated in dependent claims and are described in detail below.

The catheter system comprises an outer catheter and an inner (tubular) member.

The outer catheter has an outer catheter shaft defining a (tubular) lumen capable of receiving an inner (tubular) member. The outer catheter has a proximal outer catheter end and a distal outer catheter end. The distal outer catheter end is the end which is inserted into a human or animal body. The proximal outer catheter end is the end, which is operated by an operator, in particular an interventional physician e.g. cardiologist or radiologist.

The outer catheter may be a support catheter.

The inner (tubular) member and the outer catheter (e.g. support catheter) are relatively movable to each other in an unrestricted moving state in axial and (in a very limited manner) in radial direction. Thus, in the unrestricted moving state an axial movement of the inner member is enabled.

The catheter system comprises an axial movement restriction element. The axial movement restriction element enables a restriction of the axial movement of the at least one inner member in a restricted moving state compared to the axial movement in the unrestricted moving state.

The axial movement restriction element comprises a locking mechanism and an actuation mechanism. The locking mechanism and the actuation mechanism are connected with each other or coupled to each other. Thus, the locking mechanism and the actuation mechanism are working together. The axial movement restriction element can comprise a locking mechanism element comprising the locking mechanism and an actuation mechanism element comprising the actuation mechanism, wherein the locking mechanism element and the actuation mechanism element are connected with each other or coupled to each other.

The axial movement restriction element also enables an unrestricted axial movement of the inner member in the unrestricted moving state with respect to the outer catheter (e.g. support catheter). It is to be understood by a skilled person that the unrestricted axial movement of the inner member is nevertheless limited by the catheter design.

The locking mechanism if taken individually would enable a locking of the inner member in the restricted moving state with respect to the outer catheter (e.g. support catheter) in a selectable position and the axial actuation mechanism if taken individually would enable an axial movement of the (at least one) inner member and the outer catheter relative to each other in the restricted moving state. Thus, the locking mechanism and the actuation mechanism together (or clearly spoken the movement restriction element) enable a restricted axial movement of the (at least one) inner member and the outer catheter relative to each other in the restricted moving state. Therefore, the movement restriction element comprising the locking mechanism and the actuation mechanism enables a restricted axial movement of the (at least one) inner member and the outer catheter relative to each other in the restricted moving state. The axial movement can be a simple displacement (i.e. a translatory movement) or a combined rotatory and translatory movement, preferably a regular or irregular oscillatory movement.

A catheter system comprising
- an outer catheter having an outer catheter shaft defining an outer catheter lumen capable of receiving (at least) one inner member,
- (at least) one inner member being arranged within the outer catheter lumen of the outer catheter, the (at least one) inner member being relatively movable to the outer catheter in an unrestricted moving state, and
- an axial movement restriction element comprising an actuation mechanism and a locking mechanism, wherein the axial movement restriction element is capable of restricting an axial movement of the (at least one) inner member in a restricted moving state compared to the axial movement in the unrestricted moving state.

The (at least one) inner member preferably is an inner tubular member or a wire-shaped inner member (or a combination thereof). The outer catheter lumen preferably is a tubular outer catheter lumen.

The combination of the locking mechanism and the actuation mechanism enables a variable usable length of the inner (tubular) member during operability.

The support catheter has a support catheter shaft defining a (tubular) lumen capable of receiving an inner (tubular) member. The support catheter has a proximal support catheter end and a distal support catheter end. The distal support catheter end is the end which is inserted into a human or animal body. The proximal support catheter end is the end, which is operated by an operator, in particular an interventional physician e.g. cardiologist or radiologist. The distal support catheter end may be straight-edged. A straight-edged distal support catheter end improves the pushability and avoids flaring.

The outer catheter, e.g. the support catheter, may be connected at its proximal end to an operational handle, preferably a handle enabling a pushing by an operator (namely a pushing handle). The handle may include visual, acoustic or haptic markings for improved length adjustability and handling by operators. The handle may have a gripping surface. The handle may be configured to simultaneously attach to the support catheter and the dilator.

The outer catheter, e.g. the support catheter, may further comprise a manifold member which can be connected to the operational handle, for example via a Luer connector. The manifold may have a shark fin shape.

The support catheter may further comprise one or more ports, e.g. flushing port(s), inflation and/or deflation port(s).

The catheter system may comprise as inner tubular member or in addition a dilator and/or a balloon catheter, e.g. a percutaneous transluminal angioplasty (PTA) catheter or a percutaneous transluminal coronary angioplasty (PTCA) catheter, and/or a catheter for applying a fluid medium, e.g. a contrast agent or a fluid comprising a drug, and/or a coronary catheter or a peripheral catheter (catheter for radiology interventions) and/or an otherwise interventional catheter.

The catheter system may comprise as wire-shaped inner member or in addition a needle or a guidewire.

The catheter system may be a multi-functional catheter system or interventional catheter system. Multi-Functional catheter system means that the support catheter can be accommodated simultaneously or consecutively with different inner members, like dilator and balloon catheter (e.g. **PTA** or PTCA catheter).

Preferably the catheter system comprises
- a support catheter having a support catheter shaft defining a support catheter lumen capable of receiving a dilator and/or a balloon catheter, and
- the dilator and/or the balloon catheter being arranged within the support catheter lumen of the support catheter, the dilator and/or the balloon catheter being relatively movable to the support catheter in an unrestricted moving state, and
- an axial movement restriction element comprising an actuation mechanism and a locking mechanism, wherein the axial movement restriction element is capable of restricting an axial movement of the dilator and/or the balloon catheter in a restricted moving state compared to the axial movement in the unrestricted moving state.

The support catheter, the dilator and the balloon catheter (e.g. PTA or PTCA catheter) are each dimensionally adapted to each other.

The dilator has a dilator distal end, a dilator proximal end and a dilator shaft extending between the dilator distal end and the dilator proximal end. The dilator distal end comprises a dilator tip, preferably made of a polymeric material. The tip can be a sharp or a blunt tip. The proximal dilator end may comprise a dilator manifold.

The balloon catheter has a balloon catheter distal end, a balloon catheter proximal end and a balloon catheter shaft extending between the balloon catheter distal end and the balloon catheter proximal end. The balloon catheter distal end comprises a balloon (which is an inflatable member). The proximal balloon catheter end may comprise a balloon catheter manifold. The balloon may comprise radiopaque markers.

The catheter system may comprise a guidewire. A catheter system of this kind can be guided or pushed to the location of interest with the aid of the guide wire.

The axial movement restriction element comprising the actuation mechanism and the locking mechanism can be situated at or near the proximal outer catheter end, e.g. support catheter end. The axial movement restriction element comprising the actuation mechanism and the locking mechanism may be integrated in the handle. In such a case the handle is among its other functionalities used for restricting or unrestricting the inner member of the catheter system.

The axial movement restriction element comprising the actuation mechanism and the locking mechanism can be a separate element which can be combined with any catheter system having an outer catheter and an inner member. Thus, the axial movement restriction element comprising an actuation mechanism and a locking mechanism is herein disclosed as well as an individual embodiment. The individual features of the locking mechanism and the actuation mechanism are described in detail elsewhere in the application.

In one embodiment the axial movement restriction element comprises a locking mechanism element comprising the locking mechanism and an actuation mechanism element comprising the actuation mechanism, wherein the locking mechanism element and the actuation mechanism element are connected with each other or coupled to each other. The axial movement restriction element is capable of restricting an axial movement of at least one inner member, which can be inserted in the locking mechanism (element). The axial movement restriction element or the actuation mechanism element of the axial movement restriction element comprises a connector (e.g. a Luer connector) for connecting the axial movement restriction element with an outer catheter (e.g. a support catheter) having a tubular outer catheter lumen capable of receiving an inner (tubular or wire-shaped) member. The axial movement restriction element comprises a (tubular) reception for the inner (tubular or wire-shaped) member, e.g. a catheter or a guidewire, or the locking mechanism element and the actuation mechanism element of the axial movement restriction element comprise a reception for an inner (tubular or wire-shaped) member, e.g. a catheter or a guidewire.

The individual features of the locking mechanism (element) and the actuation mechanism (element) are described in detail elsewhere in the application.

Various embodiments of the locking mechanisms or locking mechanism elements are possible. The locking mechanism can be situated in the locking mechanism element, wherein the locking mechanism element is connected with or coupled to the actuation mechanism element.

The locking mechanism can comprise at least one clamping element for locking the axial movement of the inner member in the locking state. In one embodiment the locking mechanism element can comprise at least one clamping element (suitable) for locking the axial movement of the inner member in the locking state.

In one embodiment the at least one clamping member can be a tapered clamp. Tapered clamps are pressed onto the inner member by means of a screw-in-cap that is connected via thread to the actuation mechanism (element). The tapered clamps may be designed as two half shell clamps together fixing the inner member, preferably leaving open a channel for fluid flow when pressed onto the inner member.

In another embodiment the at least one clamping member can be a flexible clamping sleeve. The flexible clamping sleeve can be pressed onto the inner member by means of an outer cap. The sleeve flexibility might be created by material behavior and/or by slotted design.

In a further embodiment the at least one clamping member can have a clip-on design.

The clip-on design can consist of two parts together fixing the inner member.

In one embodiment the locking mechanism element can be a hemostatic valve (also referred to as hemostasis valve). A hemostasis valve has a seal, which can be locked or unlocked, for example each time the inner member is introduced or extracted. The hemostatic valve may be connected to the actuation mechanism element, for example via a Luer connection.

Various embodiments of the axial actuation mechanism are possible.

The actuation mechanism can be situated in an actuation mechanism element, wherein the actuation mechanism element is connected with or coupled to the locking mechanism element.

In one embodiment the axial actuation mechanism being based on a spring-loaded actuation mechanism is preferably excluded.

In another embodiment the axial actuation mechanism can be based on a gas pressure spring.

In another embodiment the axial actuation mechanism can be based on a magnetic actuation mechanism, optionally combined with a damping element

In another embodiment the axial actuation mechanism is situated in a handle having a (lateral) lever. Via the lever an axial (forth and back) movement of the inner member can be achieved. The movement of the lever induced by the operators hand or finger(s), (e.g. a forth and back movement) can be transmitted into an axial movement of the inner member.

In another embodiment the axial actuation mechanism is situated in a handle having a (lateral) eyelet for the operators thumb or finger to (directly or indirectly) apply a movement, (e.g. a forth and back movement) of the operators thumb or finger to the inner member, e.g. using stroke limiting elements.

The axial actuation mechanism can be a manual actuation mechanism. In a manual actuation mechanism, the axial actuation is manually applied by the operator supported by defined end stops to limit the stroke. The outer catheter is spatially kept in place by the operator's one hand while the inner member is axially moved back (towards the proximal outer catheter end) and forth (towards the distal outer catheter end) by means of the operator's other hand.

Alternatively, the axial actuation mechanism can be an electrically driven actuation mechanism, preferably being initiated by the operator.

The axial actuation mechanism can be a mechanism turning a rotation into axial translation or into a combined axial translation and rotation.

**In** one embodiment the axial actuation mechanism can comprise a threaded spindle. The threaded spindle turns rotation into axial translation with pitch and stroke according to requirements (axial movement limited by design of the thread) and back and forth with bidirectional rotation or unidirectional rotation. The axial movement can result from the rotation manually applied by the operator.

In another embodiment the axial actuation mechanism can be ball screw drive actuation mechanism. A ball screw drive actuation mechanism comprises a bearing ball kept in place by an eyelet or slot in a housing of the actuation mechanism element, combined with a thread-like groove where the bearing ball forces the outer catheter comprising the groove to axially travel in the intended manner.

The inner member might rotate during that actuation or not (depending on the catheter system requirements).

The combination of each aforementioned locking mechanism (element) with each of the aforementioned axial actuation mechanism (element) is herein disclosed.

Further described is an axial movement restriction element for restricting an axial movement of at least one inner member of a catheter, preferably the inner member being a balloon or a tubular or wire-shaped inner member, like a guidewire or dilator.

The axial movement restriction element for restricting an axial movement of at least one inner member being arranged in (a catheter lumen of) a catheter, wherein the axial movement restriction element comprises a locking mechanism and an actuation mechanism. The locking mechanism and the actuation mechanism are connected with each other or coupled to each other.

The axial movement restriction element can comprise a locking mechanism element comprising the locking mechanism and an actuation mechanism element comprising the actuation mechanism, wherein the locking mechanism element and the actuation mechanism element are connected with each other or coupled to each other. For example, the actuation mechanism element can comprise an axial actuation mechanism which converts a rotary motion into a translatory motion of the locking mechanism element. Thus, the actuation mechanism element can comprise a rotation-translation gear for converting a rotational movement into a translatory movement.

The axial movement restriction element further comprises a reception for the inner member (e.g. a balloon or a tubular or wire-shaped inner member, like a guidewire or dilator). Preferably, the locking mechanism element and the actuation mechanism element of the axial movement restriction element comprise a reception for the inner member (e.g. a balloon or a tubular or wire-shaped inner member, like a guidewire or dilator).

The axial movement restriction element is capable of restricting an axial movement of at least one inner member, which can be inserted in the axial movement restriction element and/or the locking mechanism element.

The axial movement restriction element and/or the actuation mechanism element of the axial movement restriction element can comprise a connector for connecting the axial movement restriction element and/or the actuation mechanism element with a (outer) catheter having a (tubular outer) catheter lumen capable of receiving an inner member (e.g. a balloon or a tubular or wire-shaped inner member, like a guidewire or dilator). The connector may be a Luer connector.

Furthermore, a method (not claimed) for operating a catheter system as described above is described.

The method for operating a catheter system comprises
- an outer catheter having an outer catheter shaft defining an outer catheter lumen capable of receiving (at least) one inner member, and
- (at least) one inner member arranged within the outer catheter lumen of the outer catheter,
- restricting the axial movement of the at least one inner member via an axial movement restriction element comprising an actuation mechanism and a locking mechanism in a restricted moving state compared to an unrestricted moving state.

By restricting the axial movement of the (at least one) inner member a better performance can be achieved.

The method can further comprise a step of unrestricting the axial movement of the (at least one) inner member via the axial movement restriction element in the unrestricted moving state and optionally moving the inner member within the outer catheter lumen of the outer catheter.

Also described is a catheter system as described above or a method (not claimed) for operating a catheter system wherein:
- the (at least one) inner member can be an inner tubular member or an inner wire-shaped member;
- the outer catheter can be a support catheter and/or the (at least one) inner member can be a dilator, a balloon catheter, a catheter for applying a drug or a fluid medium a coronary catheter, a peripheral catheter and/or any other interventional catheter;
- the inner member can be a percutaneous transluminal angioplasty catheter or a percutaneous transluminal coronary angioplasty catheter;
- the locking mechanism can be a clamping mechanism for locking the (at least one) inner member comprising at least one clamp. The at least one clamp can be made up by several parts, preferably by two half shell clamps, working together as one clamp;
- the actuation mechanism is a manual actuation mechanism or an electrically driven actuation mechanism comprising a drive unit;
- the actuation mechanism can be spindle driven actuation mechanism or a bayonet mount driven actuation mechanism. Alternatively, the actuation mechanism comprises a groove and a bearing ball being capable of sliding within the groove;
- the actuation mechanism is not based on a spring-loaded actuation mechanism;
- the axial movement is a translatory movement or a combined rotatory and translatory movement;
- the actuation mechanism comprises a means for generating vibrations (e.g. having a frequency of 1 Hz to 100 kHz, preferably 5 Hz to 15 kHz, more preferably 5 Hz to 500 Hz) and a transmission means for transmitting said vibrations to the inner member;
- the (at least one) inner tubular member (axially)oscillates, preferably with frequency of 1 Hz to 100 kHz, more preferably 5 Hz to 15 kHz, most preferably 5 Hz to 500 Hz.

By using the actuation mechanism comprising a means for generating vibrations having a frequency of 5 Hz or more (and less than 100 kHz, 15 kHz or 500 Hz) mechanical manipulations clinically desired can be facilitated when addressing a stenosis or chronic total occlusion compared the inner member being moved manually by an operator.

Features and advantages of the present invention and embodiments thereof shall be explained in the following figures, wherein
- Fig. 1: shows an embodiment of a catheter system,
- Fig. 2: shows an embodiment of a support catheter,
- Fig. 3: shows an embodiment of a dilator,
- Fig. 4: shows an embodiment of a balloon catheter,
- Fig. 5: shows a schematic drawing of a catheter system comprising an axial movement restriction element,
- Fig. 6: shows another embodiment of a catheter system comprising an axial movement restriction element in a cross-sectional side view,
- Fig. 7: shows an embodiment of a locking mechanism in a cross-sectional side view,
- Fig. 8: shows an embodiment of a catheter system with a locking mechanism in a cross-sectional side view,
- Fig. 9: shows a further embodiment of a locking mechanism in a side view,
- Fig. 10: shows another embodiment of a catheter system with an axial movement restriction element in a cross-sectional side view,
- Fig. 11: shows a schematic drawing of an axial movement restriction element comprising an actuation mechanism and a locking mechanism,
- Fig. 12: shows another embodiment of an axial movement restriction element comprising in a cross-sectional side view,
- Fig. 13: shows another embodiment of a catheter system 1 comprising an axial movement restriction element 10 in a cross-sectional side view,
- Fig. 14: shows a part of the axial movement restriction element 10 of Fig. 13 in a cross-sectional view.
- Fig. 15: shows a part of the axial movement restriction element 10 of Fig. 13 in a cross-sectional front view,
- Fig. 16: shows a part of the axial movement restriction element 10 of Fig. 13 in a cross-sectional front view,
- Fig. 17A-17C: show another embodiment of an axial movement restriction element
- Fig. 18: shows the axial movement restriction element 10 as described in Fig. 13 in a cross-sectional side view.

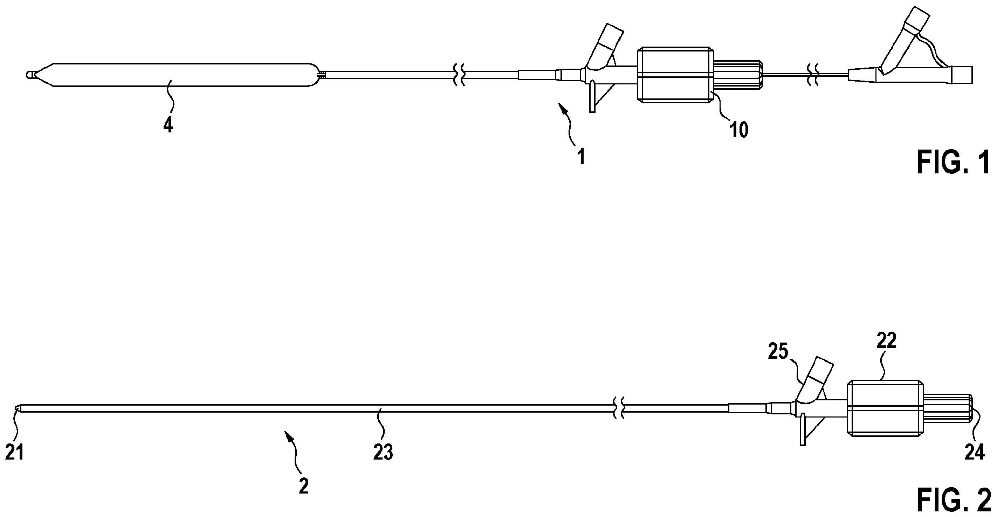

Fig. 1 shows a catheter system 1 comprising an outer catheter (e.g. a support catheter). The outer catheter shaft defines a tubular outer catheter lumen capable of receiving one inner tubular member. The catheter system 1 comprises an axial movement restriction element 10. Here the inner tubular member shown is a balloon catheter 4 (e.g. PTA catheter), which is arranged within the tubular outer catheter lumen of the outer catheter. However instead of the balloon catheter a dilator can be used instead. The inner tubular member is relatively movable to the outer catheter in an unrestricted moving state.

In Fig. 2 a support catheter 2 is shown. The support catheter 2 has a support catheter distal end 21, a support catheter proximal end 24 and a support catheter shaft 23 extending between the support catheter distal end 21 and the support catheter proximal end 24. The proximal support catheter end 24 may comprise a support catheter handle 22 being connected to a support catheter manifold 25. The manifold can comprise a port.

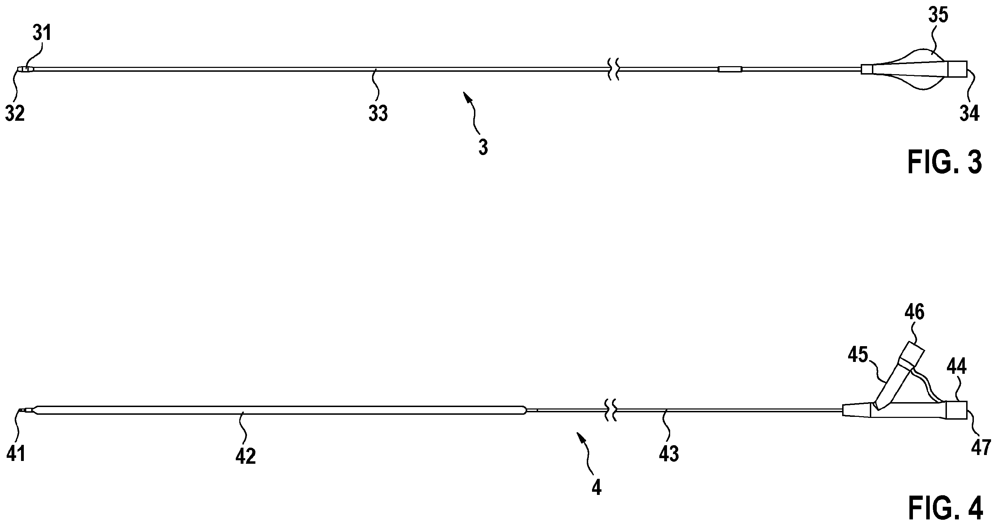

Fig. 3 shows a dilator 3 having a dilator distal end 31, a dilator proximal end 34 and a dilator shaft 33 extending between the dilator distal end 31 and the dilator proximal end 34. The dilator distal end 31 comprises a dilator tip 32. The tip can be a sharp or a blunt tip. The proximal dilator end 34 may connected with a dilator manifold 25.

Fig. 4 shows a balloon catheter 4 having a balloon catheter distal end 41, a balloon catheter proximal 44 end and a balloon catheter shaft 43 extending between the balloon catheter distal end 41 and the balloon catheter proximal end 44. The balloon catheter distal end 41 comprises a balloon 42 (which is an inflatable member). The proximal balloon catheter end 44 may comprise a balloon catheter manifold 45. The manifold may comprise an inflation/deflation port 46 and a guidewire port 47.

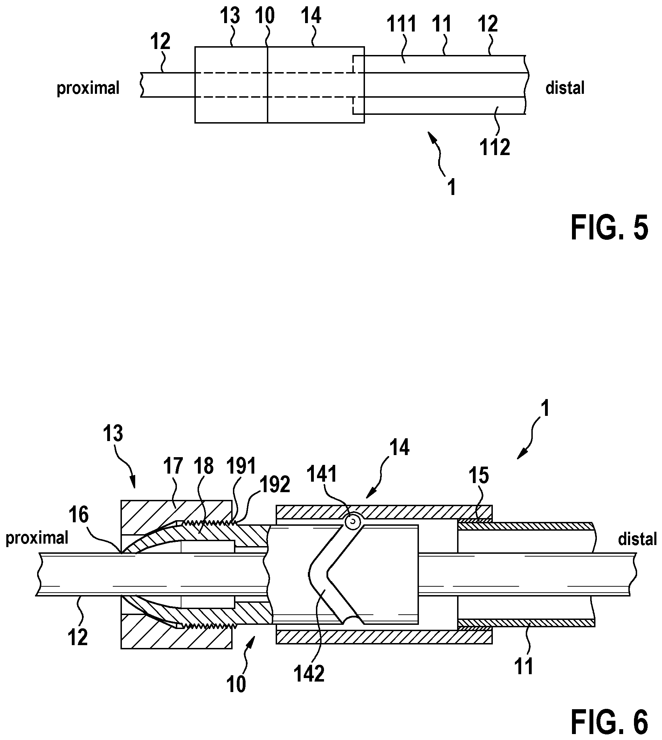

Fig. 5 shows a catheter system 1 comprising an outer catheter 11 having an outer catheter shaft 112 defining an outer catheter lumen 111 capable of receiving at least one inner member 12, and at least one inner member 12 being arranged within the outer catheter lumen 111 of the outer catheter 11. The catheter system 1 further comprises an axial movement restriction element 10 comprising an actuation mechanism 14 and a locking mechanism 13. The axial movement restriction element 10 is capable of restricting an axial movement of the at least one inner member 12 in a restricted moving state compared to the axial movement in the unrestricted moving state. The actuation mechanism 14 is connected to the locking mechanism 13.

Fig. 6 shows another embodiment of a catheter system 1 comprising an axial movement restriction element 10 comprising an actuation mechanism 14 and a locking mechanism 13 of the catheter system in a cross-sectional side view. The actuation mechanism comprises a bearing ball 141 kept in place by a bearing ball receptacle (e.g. an eyelet or slot) in a housing surrounding the actuation mechanism combined with a (thread-like) groove 142 where the bearing ball 141 forces the part with the groove 142 to axially travel in the intended manner. The axial movement results from the rotation manually applied by the clinical user. The groove may be an oscillating groove. The outer catheter 11 (only partly shown) is connected to the actuation mechanism 14 of the axial movement restriction element 10 via a connection element 15, e.g. a Luer connector. Preferably, the bearing ball receptacle is connected to the outer catheter 11. The outer catheter 11 (only partly shown) is spatially kept in place (users first hand) while the inner member 12 is axially propagating driven by the handle rotated by users second hand. The inner catheter might rotate during that actuation or not according to product requirements and implemented embodiment. The actuation mechanism 14 is connected to the locking mechanism 13. The locking mechanism comprises a stiff member 17 having a first thread part 191 and a sleeve 18 having a second thread part 192. The sleeve has 18 a soft tip 16, e.g. a tip made of an elastomer.

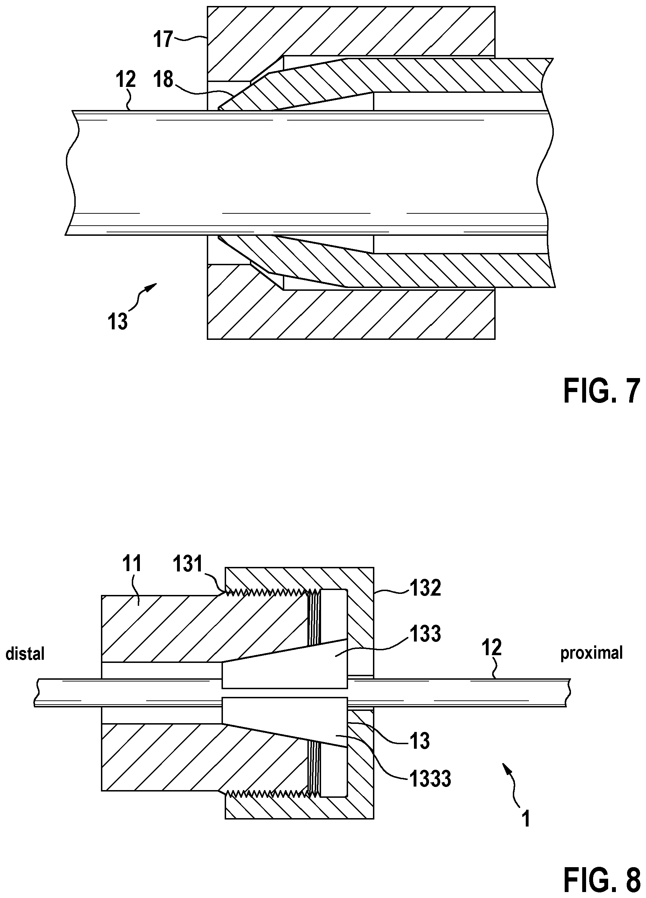

Fig. 7 shows an embodiment of a locking mechanism 13 in a longitudinal cross-sectional side view. The at least one clamping member can comprise a flexible clamping sleeve 18. The flexible sleeve is pressed onto the inner member 12 by means of an outer cap 17.

Fig. 8 shows an embodiment of a catheter system 1 with a locking mechanism 13 in a cross-sectional side view. The at least one clamping member 133 can be a tapered clamp. The clamping member 133 may be made of an elastomer. The tapered clamp is pressed onto the inner member 12 by means of a screw-in-cap 132 that is connected via thread 131 to the outer catheter shaft 11. The tapered clamp may be designed as two half shell clamps 1333. The two half shell clamps 1333, working together as one clamp, can be pressed onto the inner member 12 by means of the screw-in-cap 132. The two half shell clamps leave an open channel for fluid flow when pressed onto the inner member 12. The half shell clamps may be directly connected to the screw-in-cap 132 or are formed integrally with the screw-in-cap 132.

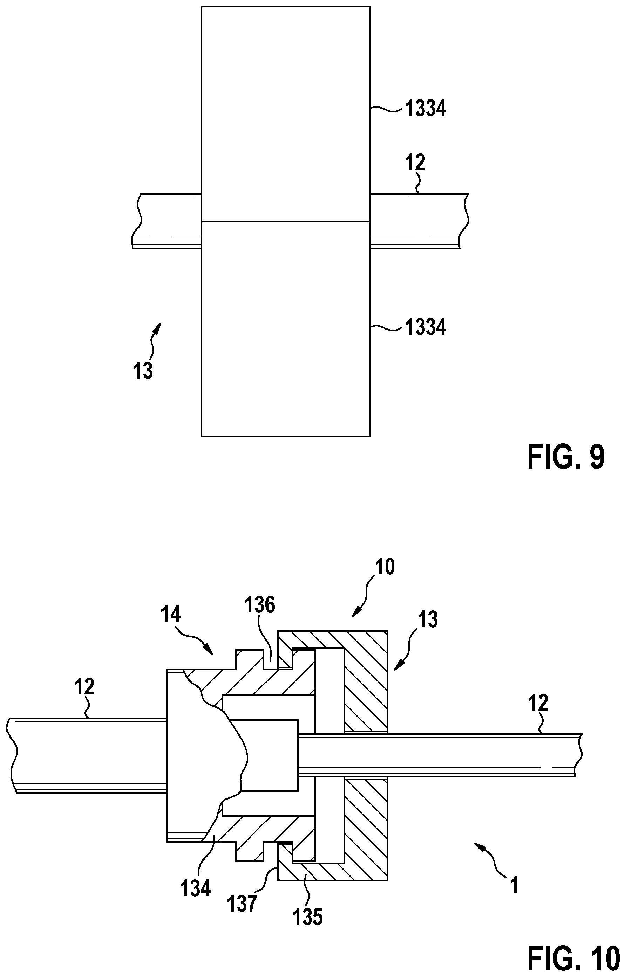

Fig. 9 shows a further embodiment of a locking mechanism 13 in a side view which is a clip-on mechanism with two clip-on half shells 1334 pressed onto the inner member 12. The releasable clip-on mechanism based on e.g. frictional forces or a snap hook design. The clip-on half shells may comprise an elastomer on the side of the half shells facing the inner member 12.

Fig. 10 shows another embodiment of a catheter system 1 with an axial movement restriction element 10 comprising an actuation mechanism 14 and a locking mechanism 13 in a side view. The axial actuation mechanism 14 and the locking mechanism 13 are connected to each other and work together. The axial actuation mechanism 14 is manually applied by the clinical user supported by defined end stops to limit the stroke. The outer catheter 11 is spatially kept in place (by user's first hand) while the inner member 12 is axially propagating back and forth by means of user's second hand. The locking mechanism 13 comprises of a two-part stroke comprising a first part of an axial two way stroke limiter 134 comprising a depression 136 and a second part of an axial two way stroke limiter 135 (as counterpart) comprising a protrusion 137 working together with the groove 136 of the first part of an axial two way stroke limiter 134. The second part of an axial two-way stroke limiter 135 has a circumferential inner ledge engaging with the first part of an axial two-way stroke limiter 134 e.g. via a groove to limit axial stroke in two directions. The first part of the axial two-way stroke limiter 134 is connected to or integral part of the outer catheter 11. The second part of the axial two-way stroke limiter 135 is connected to the inner member 12. The locking mechanism 13 is formed integrally with the second part of an axial two-way stroke limiter 135.The locking mechanism is releasably being clamped or snapped on the inner catheter 12 preferably during clinical procedure.

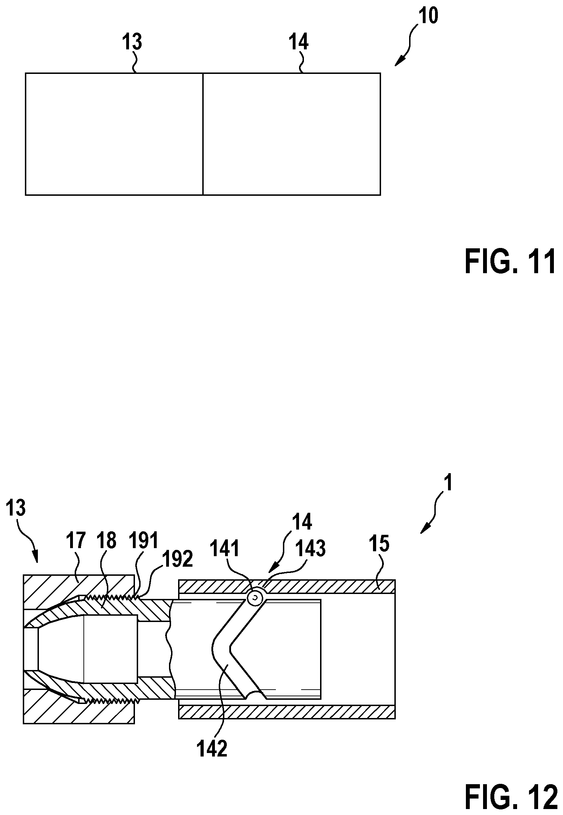

Fig. 11 shows an embodiment of an axial movement restriction element 10 comprising an actuation mechanism 14 and a locking mechanism 13. The actuation mechanism 14 is connected to the locking mechanism 13. The axial movement restriction element 10 can comprise a locking mechanism element comprising the locking mechanism 13 and an actuation mechanism element comprising the actuation mechanism 14, wherein the locking mechanism element and the actuation mechanism element are connected with each other or coupled to each other. The axial movement restriction element 10 is capable of restricting an axial movement of at least one inner member, which can be inserted in the locking mechanism (element). The axial movement restriction element 10 or the actuation mechanism element of the axial movement restriction element 10 may comprise a connector for connecting the axial movement restriction element 10 with an outer catheter (e.g. a support catheter) having an outer catheter lumen capable of receiving an inner (tubular or wire-shaped) member. The axial movement restriction element 10 comprises a reception for the inner (tubular or wire-shaped) member, e.g. a catheter or a guidewire. Preferably the locking mechanism element and the actuation mechanism element of the axial movement restriction element 10 comprise a reception for an inner (tubular or wire-shaped) member, e.g. a catheter or a guidewire.

Fig. 12 shows another embodiment of an axial movement restriction element comprising an actuation mechanism 14 and a locking mechanism 13 in a cross-sectional side view. The actuation mechanism 14 comprises a bearing ball 141 kept in place by bearing ball receptacle 143 (e.g. eyelet or slot) situated in an actuation mechanism housing which is combined with a thread-like groove 142 where the bearing ball 141 forces the part with the groove 142 to axially travel in the intended manner. The axial movement results from the rotation applied by an operator. The groove may be an oscillating groove. The actuation mechanism 14 of the axial movement restriction element 10 can be connectable to an outer catheter (not shown) via a connection element 15, e.g. a Luer connector. Preferably, the bearing ball receptacle 143 is connectable to the outer catheter. The actuation mechanism 14 is connected to the locking mechanism 13. The locking mechanism comprises a stiff member 17 having a first thread part 191 and a sleeve 18 having a second thread part 192. The sleeve has a soft tip 16, e.g. a tip made of an elastomer.

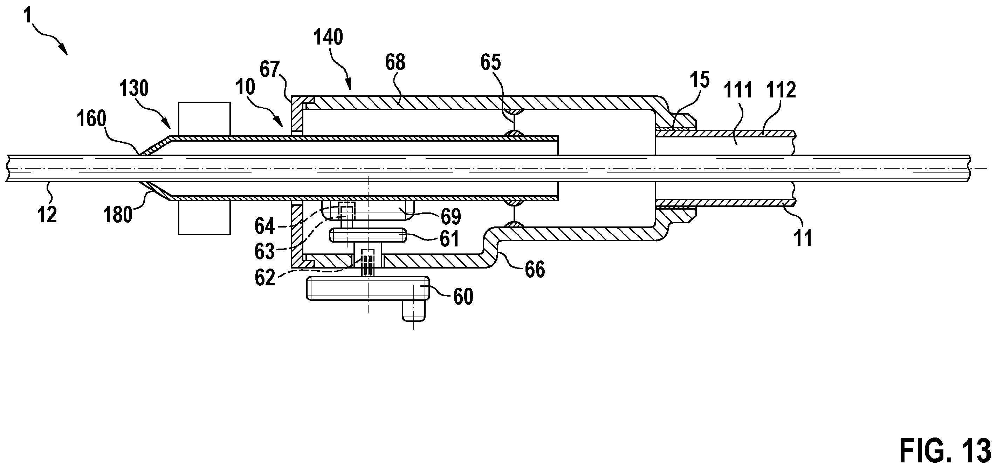

Fig. 13 shows another embodiment of a catheter system 1 comprising an axial movement restriction element 10 in a cross-sectional side view. The axial movement restriction element 10 comprises a locking mechanism element 130 comprising a locking mechanism and an actuation mechanism element 140 comprising an actuation mechanism, wherein the locking mechanism element 130 and the actuation mechanism element 140 are connected with each other or coupled to each other and interact with each other. Thus, the actuation mechanism is coupled to the locking mechanism. The actuation mechanism element 140 comprises an axial actuation mechanism. In the axial actuation mechanism shown in Fig. 13 a rotary motion is converted into a translatory motion of the locking mechanism element 130. The actuation mechanism element 140 may comprise a rotation-translation gear for converting a rotational movement into a translatory movement. However, the actuation mechanism element 140 could also comprise an axial actuation mechanism, wherein a translational movement is converted directly or indirectly into a translatory movement.

The catheter system 1 comprises an outer catheter 11 (e.g. a guiding catheter) having an outer catheter shaft 112 which defines a tubular outer catheter lumen 111 capable of receiving a (tubular) inner member 12 (e.g. a dilator). The axial movement restriction element 10, specifically the actuation mechanism element 140, is connected via a connection element 15 (e.g. a Luer connector) with the outer catheter 11.

In detail, the locking mechanism element 130 may comprise a sleeve 180. The sleeve 180 may comprise a flexible tip 160 at its proximal sleeve part. The sleeve 180 may comprise a seal 65 at its distal sleeve part. The seal 65 enables an axial lifting movement. The seal 65, e.g. a sealing ring, a sealing membrane or sealing lip, may have a sealing function for a fluid and at the same time acts as a guiding element. The sleeve 180 may further comprise a notch 64 in its middle sleeve part, being situated between the proximal sleeve part and the distal sleeve part or the notch may be situated at a notch element 69 (e.g. disk) being directly attached to the middle sleeve part. The notch 64 may have a spiral track for adjusting the axial lifting movement. The locking mechanism element 130 and the actuation mechanism element 140 are coupled to each other via the seal 65.

The actuation mechanism element 140 may comprise a first rotating element 60, a drive shaft 62 (with or without gear transmission), a second rotating element 61 and a housing 66. The housing 66 may be a two-part housing comprising a main housing body 68 and a housing cap 67. The main housing body 68 and a housing cap 67 can be assembled, e.g. clipped together. The housing cap 67 can act as guiding support for the sleeve 18.

The first rotating element 60 is preferably a rotary wheel or a crank (being operable with one finger or thumb). The second rotating element 61 is preferably smaller than the first rotating element 60. The second rotating element 61 may comprise a pin 63. The first rotating element 60 can be located (partially) inside or outside the housing 66.

The movement of the first rotating element 60 is transferred via the drive shaft 62 with or without gear transmission to the second rotating element 61. The pin 63 of the second rotating element 61 interacts with the notch 64 and thus causes a translatory movement of the sleeve 18. Thus, the axial actuation of this axial movement restriction element 10 is triggered by converting a rotary motion of the first rotating element 60, e.g. a rotary wheel or crank, of the actuation mechanism element 140 into a translatory motion of the sleeve 18. The flexible tip of the sleeve 18 is pressed onto the inner member 12 and thus the inner member is locked.

Instead of the first rotating element 60, the drive shaft 62 and the second rotating element 61 with the pin 63 interacting with the notch 64 the actuation mechanism element 140 can comprise any actuation mechanism (excepting spring based mechanisms) converting a rotary or translatory movement into a translatory motion of the sleeve 18. This can be for example a mechanism where an operator needs to press a button or operate a lever.

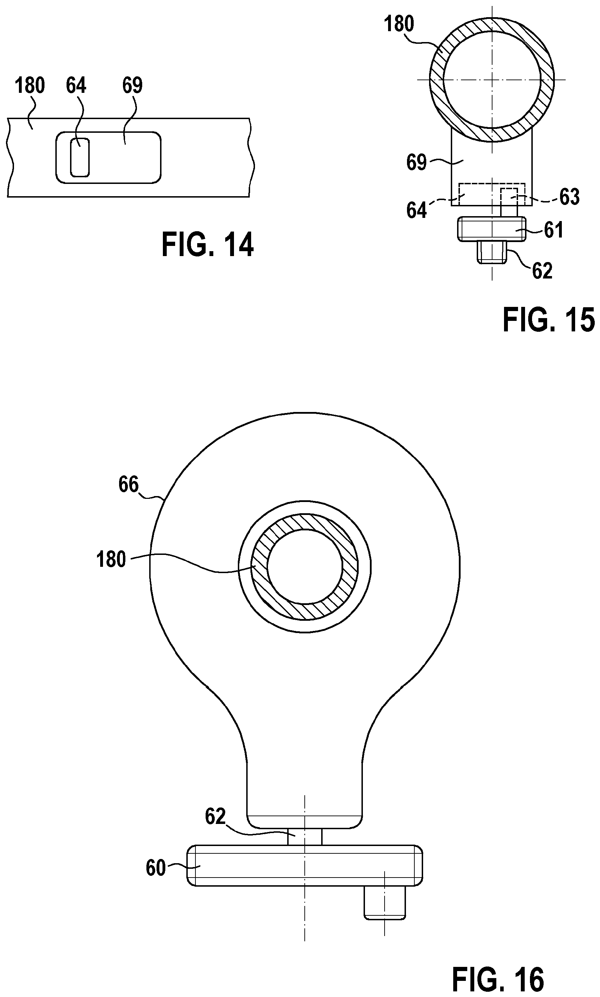

Fig. 14 shows a part of the axial movement restriction element 10 of Fig. 13 in a cross-sectional view. This part shows the sleeve 180 being connected or coupled to the notch element 69 (e.g. disk) comprising the notch 64.

Fig. 15 shows a part of the axial movement restriction element 10 of Fig. 13 in a cross-sectional front view. This part shows the sleeve 180 being connected or coupled to the notch element 69 (e.g. disk) comprising the notch 64. The notch 64 interacts with the pin 63, which is mounted on the second rotating element 61.

Fig. 16 shows a part of the axial movement restriction element 10 of Fig. 13 in a cross-sectional front view. This part shows the sleeve 180 being mounted in the housing 66. Here a part of the drive shaft 62 and the first rotating element 60 is located outside the housing. Alternatively, the drive shaft 62 can be located within the housing 66. Alternatively, a part of or the whole first rotation element 60 can be located within the housing 66, then an opening for a finger or thumb of the operator is necessary within the housing 66 through which the first rotation element 60 can be controlled.

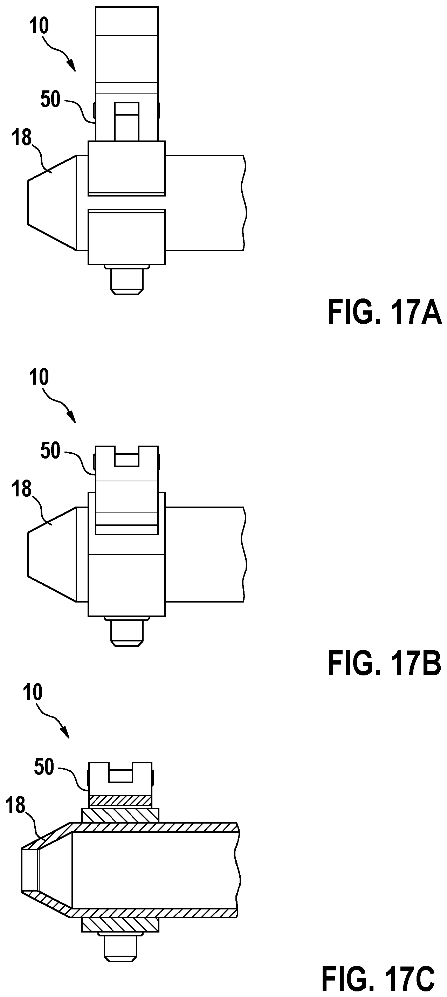

Fig. 17A, 17B and 17C show another embodiment of an axial movement restriction element 10 comprising a force application element 50 which is configured to apply a force to the sleeve 18 in circumferential direction. By applying a circumferential force to the sleeve 18 the axial movement restriction element 10 is capable of restricting an axial movement of the at least one inner member in a restricted moving state compared to the axial movement in an unrestricted moving state. The force application element 50 may be a ring-shaped or tubular-shaped force application element 50. The force application element 50 may be a quick release clamp e.g. comprising a ring-shaped or tubular-shaped collar. The type of mechanism used by the quick-release clamp can comprise a lever, a trigger, or a spring. A quick-release clamp is configured to be used one-handed. Fig. 17A shows a side view of the axial movement restriction element 10, wherein the force application element 50 is in an unrestricted moving state, for example a lever which is fastened by pushing it towards the ring-shaped collar of the quick-release clamp. Fig. 17B shows a side view of the axial movement restriction element 10, wherein the force application element 50 is in a restricted moving state, for example a lever which is loosened by pulling the lever away from the ring-shaped collar of the quick-release clamp. Fig. 17 shows a cross sectional side view of the axial movement restriction element 10 comprising a sleeve 18 and a force application element 50.

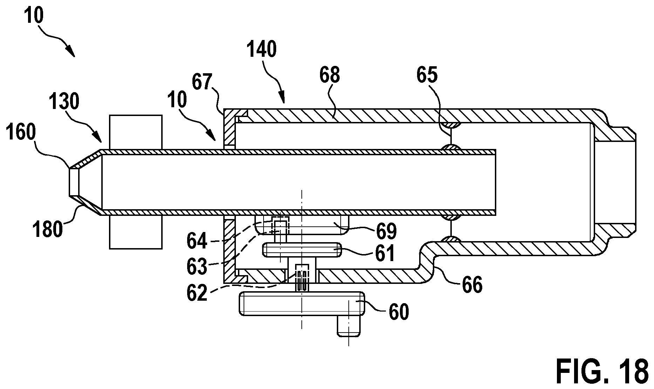

Fig. 18 shows the axial movement restriction element 10 as described in Fig. 13 in a cross-sectional side view. The axial movement restriction element 10 comprises a locking mechanism element 130 comprising a locking mechanism and an actuation mechanism element 140 comprising an actuation mechanism, wherein the locking mechanism element 130 and the actuation mechanism element 140 are connected with each other or coupled to each other. Thus, the actuation mechanism is coupled to the locking mechanism. The actuation mechanism element 140 comprises an axial actuation mechanism which converts a rotary motion into a translatory motion of the locking mechanism element 130. Preferably, the actuation mechanism element 140 comprises a rotation-translation gear.

### List of Reference signs

- 1: catheter system
- 10: axial movement restriction element
- 11: outer catheter
- 111: outer catheter lumen
- 112: outer catheter shaft
- 12: inner member
- 13: locking mechanism
- 130: locking mechanism
- 131: thread
- 132: screw in cap
- 133: clamping member
- 1333: half shell clamp
- 1334: clip-on half shells
- 134: first part of axial two-way stroke limiter
- 135: second part of two-way stroke limiter
- 136: depression
- 137: protrusion
- 14: Actuation mechanism
- 140: Actuation mechanism
- 141: bearing ball
- 143: bearing ball receptacle
- 142: groove
- 15, 150: connection element
- 16, 160: tip
- 17: stiff member
- 171: cap
- 18, 180: sleeve
- 191: first thread part
- 192: second thread part
- 2: support catheter
- 21: support catheter distal end
- 22: support catheter handle
- 23: support catheter shaft
- 24: support catheter proximal end
- 25: support catheter manifold
- 3: dilator
- 31: dilator distal end
- 32: dilator tip
- 33: dilator shaft
- 34: catheter proximal end
- 35: dilator manifold
- 4: balloon catheter
- 41: balloon catheter distal end
- 42: balloon
- 43: balloon catheter shaft
- 44: balloon catheter proximal end
- 45: balloon catheter manifold
- 46: inflation/deflation port
- 47: guidewire port
- 50: force application element
- 60: first rotating element
- 61: second rotating element
- 62: drive shaft
- 63: pin
- 64: notch
- 65: seal
- 66: housing
- 67: housing cap
- 68: main housing body
- 69: notch element

## Claims

1. A catheter system (1) comprising or consisting of:
- an outer catheter (11) having an outer catheter shaft (112) defining an outer catheter lumen (111) capable of receiving at least one inner member (12),
- at least one inner member (12) being arranged within the outer catheter lumen (111) of the outer catheter (11), the at least one inner member (12) being relatively movable to the outer catheter (11) in an unrestricted moving state, and
- an axial movement restriction element (10) comprising an actuation mechanism (14) and a locking mechanism (13), wherein the axial movement restriction element (10) is capable of restricting an axial movement of the at least one inner member (12) in a restricted moving state compared to the axial movement in the unrestricted moving state, and wherein the actuation mechanism (14) comprises a rotation-translation gear or comprises a ball screw drive actuation mechanism or the actuation mechanism (14) is a spindle driven actuation mechanism.

2. The catheter system of claim 1, wherein the actuation mechanism (14) and the locking mechanism (13) are connected with each other or coupled to each other.

3. The catheter system of claim 1 or 2, wherein the axial movement restriction element (10) comprises a connector for connecting the axial movement restriction element (10) with the outer catheter (11).

4. The catheter system of any of the preceding claims, wherein the axial movement restriction element (10) comprises a reception for the inner member (12).

5. The catheter system of any of the preceding claims, wherein the outer catheter (11) is connected at its proximal end to a handle.

6. The catheter system of claim 5, wherein the axial movement restriction element (10) is integrated in the handle.

7. The catheter system of any of the preceding claims, wherein the outer catheter (11) is a support catheter.

8. The catheter system of any of the preceding claims, wherein the at least one inner member (12) is an inner tubular member or an inner wire-shaped member.

9. The catheter system of any of the preceding claims, wherein the at least one inner member (12) is a dilator and/or a balloon catheter, preferably a percutaneous transluminal angioplasty catheter, or a catheter for applying a drug or a fluid medium or a catheter for radiology or cardiology interventions.

10. The catheter system of any of the preceding claims, wherein the locking mechanism (13) is capable of restricting the axial movement of the at least one inner member (12).

11. The catheter system of any of the preceding claims, wherein the locking mechanism (13) is a clamping mechanism for locking the at least one inner member (12) comprising at least one clamp, preferably the at least one clamp is made up by several parts, preferably by two half shell clamps, working together as one clamp.

12. The catheter system of any of the preceding claims, wherein the actuation mechanism (14) is a manual actuation mechanism or the actuation mechanism (14) is an electrically driven actuation mechanism comprising a drive unit or the actuation mechanism (14) comprises a ball screw drive actuation mechanism, which comprises an oscillating thread.

13. Axial movement restriction element (10) for restricting an axial movement of at least one inner member (12) being arranged in an outer catheter lumen (111) of an outer catheter (11), wherein the axial movement restriction element (10) comprises or consists a locking mechanism (13) and an actuation mechanism (14), wherein the locking mechanism (13) and the actuation mechanism (14) are connected with each other or coupled to each other and wherein the axial movement restriction element (10) comprises a connector for connecting the axial movement restriction element (10) with an outer catheter (11) having a tubular outer catheter lumen capable of receiving an inner member (12) and wherein the axial movement restriction element (10) comprises a reception for the inner member (12), and wherein the actuation mechanism (14) comprises a rotation-translation gear or comprises a ball screw drive actuation mechanism or the actuation mechanism (14) is a spindle driven actuation mechanism.

14. The axial movement restriction element (10) of claim 13, wherein the axial movement restriction element (10) is not attached to or connected to a catheter, preferably the outer catheter (11) or the inner member (12).

15. The catheter system (1) of claim 1 for use in a method of treating stenosis or chronic total occlusion or for use in a method of guide-wire negotiation, lesion penetration or lesion recanalization or for use in a method of applying at least one drug or a fluid medium.

## Patentansprüche

1. Kathetersystem (1), umfassend oder bestehend aus:
- einem Außenkatheter (11) mit einem Außenkatheterschaft (112), der ein Außenkatheterlumen (111) definiert, das geeignet ist, mindestens ein Innenglied (12) aufzunehmen,
- mindestens einem Innenglied (12), das innerhalb des Außenkatheterlumens (111) des Außenkatheters (11) angeordnet ist, wobei das mindestens eine Innenglied (12) relativ zum Außenkatheter (11) in einem uneingeschränkten Bewegungszustand beweglich ist, und
- einem axialen Bewegungsbeschränkungselement (10), umfassend einen Betätigungsmechanismus (14) und einen Verriegelungsmechanismus (13), wobei das axiale Bewegungsbeschränkungselement (10) geeignet ist, eine axiale Bewegung des mindestens einen Innenglieds (12) in einem beschränkten Bewegungszustand im Vergleich zur axialen Bewegung im uneingeschränkten Bewegungszustand zu beschränken, und wobei der Betätigungsmechanismus (14) ein Dreh-Translations-Getriebe umfasst oder einen Kugelgewindetrieb-Betätigungsmechanismus umfasst oder der Betätigungsmechanismus (14) ein spindelgetriebener Betätigungsmechanismus ist.

2. Kathetersystem nach Anspruch 1, wobei der Betätigungsmechanismus (14) und der Verriegelungsmechanismus (13) miteinander verbunden sind oder aneinander gekoppelt sind.

3. Kathetersystem nach Anspruch 1 oder 2, wobei das axiale Bewegungsbeschränkungselement (10) einen Verbinder zum Verbinden des axialen Bewegungsbeschränkungselements (10) mit dem Außenkatheter (11) umfasst.

4. Kathetersystem nach einem der vorhergehenden Ansprüche, wobei das axiale Bewegungsbeschränkungselement (10) eine Aufnahme für das Innenglied (12) umfasst.

5. Kathetersystem nach einem der vorhergehenden Ansprüche, wobei der Außenkatheter (11) an seinem proximalen Ende mit einem Griff verbunden ist.

6. Kathetersystem nach Anspruch 5, wobei das axiale Bewegungsbeschränkungselement (10) in den Griff integriert ist.

7. Kathetersystem nach einem der vorhergehenden Ansprüche, wobei der Außenkatheter (11) ein Stützkatheter ist.

8. Kathetersystem nach einem der vorhergehenden Ansprüche, wobei das mindestens eine Innenglied (12) ein inneres rohrförmiges Glied oder ein inneres drahtförmiges Glied ist.

9. Kathetersystem nach einem der vorhergehenden Ansprüche, wobei das mindestens eine Innenglied (12) ein Dilatator und/oder ein Ballonkatheter ist, vorzugsweise ein perkutaner transluminaler Angioplastie-Katheter, oder ein Katheter zum Applizieren eines Arzneimittels oder eines Fluidmediums oder ein Katheter für radiologische oder kardiologische Interventionen.

10. Kathetersystem nach einem der vorhergehenden Ansprüche, wobei der Verriegelungsmechanismus (13) geeignet ist, die axiale Bewegung des mindestens einen Innenglieds (12) zu beschränken.

11. Kathetersystem nach einem der vorhergehenden Ansprüche, wobei der Verriegelungsmechanismus (13) ein Klemmmechanismus zum Verriegeln des mindestens einen Innenglieds (12) ist, umfassend mindestens eine Klemme, wobei vorzugsweise die mindestens eine Klemme aus mehreren Teilen gebildet ist, vorzugsweise durch zwei Halbschalenklemmen, die zusammen als eine Klemme wirken.

12. Kathetersystem nach einem der vorhergehenden Ansprüche, wobei der Betätigungsmechanismus (14) ein manueller Betätigungsmechanismus ist oder der Betätigungsmechanismus (14) ein elektrisch angetriebener Betätigungsmechanismus ist, umfassend eine Antriebseinheit, oder der Betätigungsmechanismus (14) einen Kugelgewindetrieb-Betätigungsmechanismus umfasst, der ein oszillierendes Gewinde umfasst.

13. Axiales Bewegungsbeschränkungselement (10) zum Beschränken einer axialen Bewegung von mindestens einem Innenglied (12), das in einem Außenkatheterlumen (111) eines Außenkatheters (11) angeordnet ist, wobei das axiale Bewegungsbeschränkungselement (10) einen Verriegelungsmechanismus (13) und einen Betätigungsmechanismus (14) umfasst oder daraus besteht, wobei der Verriegelungsmechanismus (13) und der Betätigungsmechanismus (14) miteinander verbunden sind oder aneinander gekoppelt sind, und wobei das axiale Bewegungsbeschränkungselement (10) einen Verbinder zum Verbinden des axialen Bewegungsbeschränkungselements (10) mit einem Außenkatheter (11) mit einem rohrförmigen Außenkatheterlumen umfasst, das geeignet ist, ein Innenglied (12) aufzunehmen, und wobei das axiale Bewegungsbeschränkungselement (10) eine Aufnahme für das Innenglied (12) umfasst, und wobei der Betätigungsmechanismus (14) ein Dreh-Translations-Getriebe umfasst oder einen Kugelgewindetrieb-Betätigungsmechanismus umfasst oder der Betätigungsmechanismus (14) ein spindelgetriebener Betätigungsmechanismus ist.

14. Axiales Bewegungsbeschränkungselement (10) nach Anspruch 13, wobei das axiale Bewegungsbeschränkungselement (10) nicht an einem Katheter befestigt ist oder mit einem Katheter verbunden ist, vorzugsweise dem Außenkatheter (11) oder dem Innenglied (12).

15. Kathetersystem (1) nach Anspruch 1 zur Verwendung in einem Verfahren zur Behandlung einer Stenose oder eines chronischen Totalverschlusses oder zur Verwendung in einem Verfahren der Führungsdraht-Navigation, Läsionspenetration oder Läsionsrekanalisation oder zur Verwendung in einem Verfahren zum Applizieren mindestens eines Arzneimittels oder eines Fluidmediums.

## Revendications

1. Système de cathéter (1) comprenant ou constitué de :
- un cathéter externe (11) ayant un corps de cathéter externe (112) définissant une lumière de cathéter externe (111) apte à recevoir au moins un élément interne (12),
- au moins un élément interne (12) disposé à l'intérieur de la lumière de cathéter externe (111) du cathéter externe (11), ledit au moins un élément interne (12) étant mobile par rapport au cathéter externe (11) dans un état de déplacement non restreint, et
- un élément de restriction de mouvement axial (10) comprenant un mécanisme d'actionnement (14) et un mécanisme de verrouillage (13), dans lequel l'élément de restriction de mouvement axial (10) est apte à restreindre un mouvement axial dudit au moins un élément interne (12) dans un état de déplacement restreint par rapport au mouvement axial dans l'état de déplacement non restreint, et dans lequel le mécanisme d'actionnement (14) comprend un mécanisme de transmission rotation-translation ou comprend un mécanisme d'actionnement à vis à billes ou le mécanisme d'actionnement (14) est un mécanisme d'actionnement entraîné par une vis d'entraînement.

2. Système de cathéter selon la revendication 1, dans lequel le mécanisme d'actionnement (14) et le mécanisme de verrouillage (13) sont reliés l'un à l'autre ou couplés l'un à l'autre.

3. Système de cathéter selon la revendication 1 ou 2, dans lequel l'élément de restriction de mouvement axial (10) comprend un connecteur destiné à relier l'élément de restriction de mouvement axial (10) au cathéter externe (11).

4. Système de cathéter selon l'une quelconque des revendications précédentes, dans lequel l'élément de restriction de mouvement axial (10) comprend une réception pour l'élément interne (12).

5. Système de cathéter selon l'une quelconque des revendications précédentes, dans lequel le cathéter externe (11) est relié à son extrémité proximale à une poignée.

6. Système de cathéter selon la revendication 5, dans lequel l'élément de restriction de mouvement axial (10) est intégré dans la poignée.

7. Système de cathéter selon l'une quelconque des revendications précédentes, dans lequel le cathéter externe (11) est un cathéter de support.

8. Système de cathéter selon l'une quelconque des revendications précédentes, dans lequel ledit au moins un élément interne (12) est un élément tubulaire interne ou un élément interne en forme de fil.

9. Système de cathéter selon l'une quelconque des revendications précédentes, dans lequel ledit au moins un élément interne (12) est un dilatateur et/ou un cathéter à ballonnet, de préférence un cathéter d'angioplastie transluminale percutanée, ou un cathéter destiné à appliquer un médicament ou un milieu fluide ou un cathéter destiné à des interventions de radiologie ou de cardiologie.

10. Système de cathéter selon l'une quelconque des revendications précédentes, dans lequel le mécanisme de verrouillage (13) est apte à restreindre le mouvement axial dudit au moins un élément interne (12).

11. Système de cathéter selon l'une quelconque des revendications précédentes, dans lequel le mécanisme de verrouillage (13) est un mécanisme de serrage destiné à verrouiller ledit au moins un élément interne (12) comprenant au moins une pince, de préférence ladite au moins une pince est constituée de plusieurs parties, de préférence de deux pinces en demi-coquille, fonctionnant ensemble comme une seule pince.

12. Système de cathéter selon l'une quelconque des revendications précédentes, dans lequel le mécanisme d'actionnement (14) est un mécanisme d'actionnement manuel ou le mécanisme d'actionnement (14) est un mécanisme d'actionnement entraîné électriquement comprenant une unité d'entraînement ou le mécanisme d'actionnement (14) comprend un mécanisme d'actionnement à vis à billes, lequel comprend un filetage oscillant.

13. Élément de restriction de mouvement axial (10) destiné à restreindre un mouvement axial d'au moins un élément interne (12) disposé dans une lumière de cathéter externe (111) d'un cathéter externe (11), dans lequel l'élément de restriction de mouvement axial (10) comprend ou est constitué d'un mécanisme de verrouillage (13) et d'un mécanisme d'actionnement (14), dans lequel le mécanisme de verrouillage (13) et le mécanisme d'actionnement (14) sont reliés l'un à l'autre ou couplés l'un à l'autre et dans lequel l'élément de restriction de mouvement axial (10) comprend un connecteur destiné à relier l'élément de restriction de mouvement axial (10) à un cathéter externe (11) ayant une lumière de cathéter externe tubulaire apte à recevoir un élément interne (12) et dans lequel l'élément de restriction de mouvement axial (10) comprend une réception pour l'élément interne (12), et dans lequel le mécanisme d'actionnement (14) comprend un mécanisme de transmission rotation-translation ou comprend un mécanisme d'actionnement à vis à billes ou le mécanisme d'actionnement (14) est un mécanisme d'actionnement entraîné par une vis d'entraînement.

14. L'élément de restriction de mouvement axial (10) selon la revendication 13, dans lequel l'élément de restriction de mouvement axial (10) n'est ni fixé ni relié à un cathéter, de préférence au cathéter externe (11) ou à l'élément interne (12).

15. Système de cathéter (1) selon la revendication 1 destiné à être utilisé dans une méthode de traitement de la sténose ou de l'occlusion totale chronique, ou destiné à être utilisé dans une méthode de négociation de fil-guide, de pénétration de lésion ou de recanalisation de lésion, ou destiné à être utilisé dans une méthode d'application d'au moins un médicament ou un milieu fluide.
